# EUROPEAN PATENT APPLICATION

(11) **EP 4 555 996 A1**
(43) Date of publication of application: **21.05.2025**
(21) Application number: 24213936.8
(22) Date of filing: 19.11.2024
(51) Int. Cl.: A61K 8/60, A61K 8/73, A61Q 7/00

(54) **GLYCYRRHIZIC ACID BACTERIAL CELLULOSE COMPOSITE FOR PROMOTING THE GROWTH AND DEVELOPMENT OF SKIN HAIR FOLLICLES**

(30) Priority: 20.11.2023 CN 202311556506
(71) Applicant: Chen, Chao-Cheng, 104 Taipei City (TW); Hong Qing Flowery Bio-tech (Guangzhou) Co., Ltd, Guangzhou City Guangdong (CN)
(72) Inventor: CHEN, Chao-Cheng, 104 Taipei City (TW); WANG, Qiong, Guangzhou City (CN); ZHENG, Si Han, Guangzhou City (CN)
(74) Representative: Epping - Hermann - Fischer

(57) **Abstract**

The present disclosure relates to a glycyrrhizic acid bacterial cellulose composite for promoting growth and development of hair follicles in skins, in particular, the glycyrrhizic acid bacterial cellulose composite for promoting growth and development of hair follicles in skin includes glycyrrhizic acid and bacterial cellulose.

## Description

### BACKGROUND

### 1. Technical Field

The present disclosure relates to a bacterial cellulose composite, especially to a glycyrrhizic acid bacterial cellulose composite and a method for preparing the same.

### 2. Description of Associated Art

Bacterial cellulose, a novel nanomaterial less applied in drug delivery, has been confirmed to have several special physical and chemical properties including biodegradability, nontoxic property, high elastic modulus, large specific surface area, low density, non-abrasiveness, ease to surface functionalization, high purity and crystallinity of chemical components, high degree of polymerization (2000 to 8000) and high hardness. Meanwhile, the bacterial cellulose is an inert material, and thus is less used directly in a study related to drug carriers. Glycyrrhizic acid is a drug commonly used in clinical and has effects of anti-inflammation and detoxication, while there is no practical application of glycyrrhizic acid for local administration.

Therefore, there is a need for treating hair loss in clinical practice.

### SUMMARY

In view of the disadvantages of prior art, the present disclosure provides a glycyrrhizic acid bacterial cellulose composite for promoting growth and development of skin hair follicles ant a method for preparing the same, and the glycyrrhizic acid bacterial cellulose composite includes glycyrrhizic acid and bacterial cellulose. The glycyrrhizic acid bacterial cellulose composite can promote the growth and development of hair follicles in skin by topic application on the skin, which is beneficial to the treatment of hair loss in clinical practice and can promote growth of hair.

In an embodiment, the bacterial cellulose is derived from *Acetobacterium Balch* and has a molecular weight of 50,000 to 2,500,000.

In an embodiment, the bacterial cellulose has 300 to 15,000 glucosyl groups.

In an embodiment, a mass ratio of the glycyrrhizic acid and bacterial cellulose is 1:0.1 to 10; preferably, the mass ratio of the glycyrrhizic acid and bacterial cellulose is 1:5.

The present disclosure further provides a method for preparing the glycyrrhizic acid bacterial cellulose composite aforementioned, including steps of: 1) mixing water and a lyophilized bacterial cellulose to obtain an aqueous bacterial cellulose; 2) dissolving glycyrrhizic acid in water to obtain an aqueous solution of glycyrrhizic acid; and 3) adding the aqueous solution of glycyrrhizic acid dropwise to the aqueous bacterial cellulose to obtain the glycyrrhizic acid bacterial cellulose composite.

In an embodiment, the aqueous bacterial cellulose and the aqueous solution of glycyrrhizic acid are each stirred to form homogeneous solutions.

In an embodiment, the stirred aqueous bacterial cellulose and the stirred aqueous solution of glycyrrhizic acid are each further subjected to an ultrasonic treatment.

In an embodiment, the adding is performed under ultrasonication.

In an embodiment, the lyophilized bacterial cellulose and water are mixed in a weight ratio of 1:10 to 15; preferably, the glycyrrhizic acid and water are mixed in a weight ratio of 1:0.5 to 1.5.

In an embodiment, the glycyrrhizic acid and bacterial cellulose in the glycyrrhizic acid bacterial cellulose composite are present at a mass ratio of 1:0.1 to 10; preferably, the glycyrrhizic acid and bacterial cellulose in the glycyrrhizic acid bacterial cellulose composite are present at a mass ratio of 1:5.

Specifically, the glycyrrhizic acid bacterial cellulose composite provided in the present disclosure is white and sticky and bears glycyrrhizic acid on the bacterial cellulose structure thereof. In the present disclosure, a material capable of significantly facilitating the growth and development of hair follicles in skin is obtained by an amphipathic property of the glycyrrhizic acid as well as a biocompatibility, a nontoxic property and a network structure of the bacterial cellulose. The most preferable dosages of the glycyrrhizic acid and bacterial cellulose can be further selected according to study result of facilitating the growth and development of hair follicles in skin in combination with the requirements for a pharmaceutic formulation, and a related topical pharmaceutic formulation can be manufactured with a feed ratio achieving the best facilitation to the growth and development of hair follicles in skin. Since the glycyrrhizic acid itself is an amphipathic compound, and the bacterial cellulose itself has properties including nontoxic property, high biocompatibility and utilization background as a cosmetic carrier, the present disclosure can be applied to the development study of a transdermal topical formulation for the treatment of hair loss and the facilitation of hair growth.

### BRIEF DESCRIPTION OF THE DRAWINGS

The embodiments of the present application will be illustrated by exemplary reference figures:
FIGs. 1A-1C show SEM (Scanning Electron Microscope) images of Comparative Example 1, Comparative Example 3 and Example 5, respectively.
FIG. 2 shows levels of glycyrrhizic acid over different time periods in Comparative Example 2, Example 1, Example 5 and Example 10.
FIG. 3 shows levels of glycyrrhizic acid at endpoints of skin permeation in Comparative Example 2, Example 1, Example 3 and Example 5.
FIGs. 4A and 4B are tissue staining images of hair follicles in the skin untreated or treated with the glycyrrhizic acid bacterial cellulose composite of Example 10. The skin before permeation (left) and the skin after permeation (right) were stained with toluidine blue and haematoxylin-eosin (HE).

### DETAILED DESCRIPTION OF THE PREFERRED EMBODIMENTS

The embodiments of the present application will be illustrated by the following embodiments, and those skilled in the art can easily understand the advantages and benefits of the present application from the content described in this specification. The present application can be practiced or applied by other different implementations, and different modifications and alternations can be made to the details of the present specification based on different views and applications without departing from the spirit described in the present application. Furthermore, all of the ranges and values mentioned herein are inclusive and combinable. Any numerical value or point, e.g., any integer, falling into a range described herein can be used as a lower or an upper limit to derive a subrange.

Glycyrrhizic acid, a drug commonly used in clinic, has effects of anti-inflammation and detoxication, which belongs to triterpenes and has an amphipathic property, and thus exhibits the features of a surfactant. The aggregations or micella of glycyrrhizic acid can form an inclusion composite of host-guest with a hydrophobic drug, which can effectively increase the solubility of the drug and prevent the drug from precipitation.

Bacterial cellulose possesses the features of biodegradability, nontoxic property, high elasticity modulus, high specific surface area, low density, non-abrasive, ease to surface functionality, high purity and crystallinity degree of chemical components, high degree of polymerization (2000 to 8000), high hardness, etc. However, the bacterial cellulose is very inert, and there is few related study in which the bacterial cellulose is applied as a topical pharmaceutical carrier for promoting the growth of hair.

Therefore, the present disclosure utilizes the amphipathic property of the glycyrrhizic acid to develop the bacterial cellulose as a topical pharmaceutical carrier, and it is shown that the glycyrrhizic acid bacterial cellulose composite can promote the growth and development of hair follicles in skin. As described above, in the present disclosure, the glycyrrhizic acid is used as delivering drug, and the bacterial cellulose is used as a carrier, so that the glycyrrhizic acid can be loaded in the network structure of the bacterial cellulose to form a new structure due to the amphipathic property of the glycyrrhizic acid, and the glycyrrhizic acid bacterial cellulose composite formed therefrom can significantly promote the growth and development of hair follicles in skin.

In one embodiment, the glycyrrhizic acid is loaded on the bacterial cellulose to form the glycyrrhizic acid bacterial cellulose composite.

In one embodiment, the bacterial cellulose is derived from *Acetobacterium Balch,* which has a molecular weight (Mw) of 50,000 to 2,500,000, a molecular weight of 100,000 to 2,500,000, a molecular weight of 500,000 to 2,500,000, a molecular weight of 1,000,000 to 2,500,000, a molecular weight of 1,500,000 to 2,500,000, a molecular weight of 2,000,000 to 2,500,000, a molecular weight of 50,000 to 2,000,000, a molecular weight of 50,000 to 1,500,000, a molecular weight of 50,000 to 1,000,000, a molecular weight of 50,000 to 500,000, or a molecular weight of 50,000 to 100,000. For example, a molecular weight of 50,000, 100,000, 150,000, 200,000, 250,000, 300,000, 350,000, 400,000, 450,000, 500,000, 550,000, 600,000, 650,000, 700,000, 750,000, 800,000, 850,000, 900,000, 950,000, 1,000,000, 1,500,000, 2,000,000, or 2,500,000. In another embodiment, the bacterial cellulose is obtained from fermenting the *Acetobacterium Balch.*

In one embodiment, the bacterial cellulose has 300 to 15,000 glucosyl groups, 1,000 to 15,000 glucosyl groups, 2,000 to 15,000 glucosyl groups, 3,000 to 15,000 glucosyl groups, 4,000 to 15,000 glucosyl groups, 5,000 to 15,000 glucosyl groups, 6,000 to 15,000 glucosyl groups, 7,000 to 15,000 glucosyl groups, 8,000 to 15,000 glucosyl groups, 9,000 to 15,000 glucosyl groups, 10,000 to 15,000 glucosyl groups, 11,000 to 15,000 glucosyl groups, 12,000 to 15,000 glucosyl groups, 13,000 to 15,000 glucosyl groups, 14,000 to 15,000 glucosyl groups, 300 to 14,000 glucosyl groups, 300 to 13,000 glucosyl groups, 300 to 12,000 glucosyl groups, 300 to 11,000 glucosyl groups, 300 to 10,000 glucosyl groups, 300 to 9,000 glucosyl groups, 300 to 8,000 glucosyl groups, 300 to 7,000 glucosyl groups, 300 to 6,000 glucosyl groups, 300 to 5,000 glucosyl groups, 300 to 4,000 glucosyl groups, 300 to 3,000 glucosyl groups, 300 to 2,000 glucosyl groups, or 300 to 1,000 glucosyl groups, such as 300, 350, 400, 450, 500, 550, 600, 650, 700, 750, 800, 850, 900, 950, 1,000, 1,500, 2,000, 2,500, 3,000, 3,500, 4,000, 4,500, 5,000, 5,500, 6,000, 6,500, 7,000, 7,500, 8,000, 8,500, 9,000, 10,000, 10,500, 11,000, 11,500, 12,000, 12,500, 13,000, 13,500, 14,000, 14,500, or 15,000 glucosyl groups. Specifically, the bacterial cellulose has a chemical general formula of (C₆H₁₀O₅)ₙ, which is a polysaccharide consisting of a linear chain (glucoside bonds) of hundreds to thousands of β- (1→4)-linked D-glucose units. In other words, the bacterial cellulose is a macromolecular polysaccharide consisting of D-glucosyl groups with β-1,4-glucoside bonds.

In one embodiment, the mass ratio of the glycyrrhizic acid to the bacterial cellulose is 1: 0.1 to 10, e.g., 1: 0.1, 1: 0.11, 1: 0.13, 1: 0.14, 1: 0.17, 1: 0.2, 1: 0.3, 1: 0.33, 1: 0.4, 1: 0.5, 1: 0.6, 1: 0.7, 1: 0.8, 1: 0.9, 1: 1, 1: 2, 1: 3, 1: 4, 1: 5, 1: 6, 1: 7, 1: 8, 1: 9, or 1: 10. Preferably, the mass ratio of the glycyrrhizic acid to the bacterial cellulose is 1: 5.

The method for preparing the glycyrrhizic acid bacterial cellulose composite provided in the present disclosure includes the following steps: 1) mixing water and a lyophilized bacterial cellulose to obtain an aqueous bacterial cellulose; 2) dissolving glycyrrhizic acid in water to obtain an aqueous solution of glycyrrhizic acid; and 3) adding the aqueous solution of glycyrrhizic acid dropwise to the aqueous bacterial cellulose to obtain the glycyrrhizic acid bacterial cellulose composite.

In one embodiment, the lyophilized bacterial cellulose and water are mixed in a weight ratio of 1: 10 to 15, e.g., a weight ratio of 1: 10, 1: 11, 1: 12, 1: 13, 1: 14, or 1: 15.

In one embodiment, the glycyrrhizic acid and water are mixed in a weight ratio of 1: 0.5 to 1.5, e.g., 1: 0.5, 1: 0.6, 1: 0.7, 1: 0.8, 1: 0.9, 1: 1.0, 1: 1.1, 1: 1.2, 1: 1.3, 1: 1.4, or 1: 1.5.

### EXAMPLES

The present application will be illustrated in detail by specific examples below which should not to be considered to limit the scope of the present application.

### Preparation Example: glycyrrhizic acid bacterial cellulose composite

Glycyrrhizic acid (93%, G810520-25g, Shanghai Macklin Biochemical Technology Co., Ltd.) was added to distilled water, and subjected to ultrasonication for 10 minutes after evenly stirred to obtain an aqueous solution of glycyrrhizic acid. Meanwhile, the bacterial cellulose derived from *Acetobacterium Balch* (nanoscale lypophilized tablet of bacterial cellulose, Evophancie Biotech Ltd.) was added to distilled water, stirred thoroughly, and subjected to ultrasonication for 10 minutes to obtain an aqueous bacterial cellulose. Under ultrasonication, the aqueous solution of glycyrrhizic acid was added dropwise at a rate of 5 drops per 10 seconds to the aqueous bacterial cellulose, stirred thoroughly, and subjected to ultrasonication for 15 minutes to obtain the glycyrrhizic acid bacterial cellulose composite.

Following the method of the aforementioned Preparation Example, the bacterial cellulose composites of Examples 1-19 were prepared by using the composition in Table 1 below, respectively, and the mass ratios of the glycyrrhizic acid to the bacterial cellulose in the prepared glycyrrhizic acid bacterial cellulose composites (products) were listed.

**Table 1: Examples 1-19**

| | Aqueous solution of glycyrrhizic acid | | Aqueous bacterial cellulose | | Mass ratio of glycyrrhizic acid and bacterial cellulose in product |
|---|---|---|---|---|---|
| | Glycyrrhizic acid (mg) | Distilled water (mL) | Bacterial cellulose (mg) | Distilled water (mL) | |
| Example 1 | 10 | 0.8 | 10 | 0.13 | 1:1 |
| Example 2 | 10 | 0.8 | 20 | 0.26 | 1:2 |
| Example 3 | 10 | 0.8 | 30 | 0.39 | 1:3 |
| Example 4 | 10 | 0.8 | 40 | 0.52 | 1:4 |
| Example 5 | 10 | 0.8 | 50 | 0.65 | 1:5 |
| Example 6 | 10 | 0.8 | 60 | 0.78 | 1:6 |
| Example 7 | 10 | 0.8 | 70 | 0.91 | 1:7 |
| Example 8 | 10 | 0.8 | 80 | 1.04 | 1:8 |
| Example 9 | 10 | 0.8 | 90 | 1.17 | 1:9 |
| Example 10 | 10 | 0.8 | 100 | 1.3 | 1:10 |
| Example 11 | 100 | 8 | 50 | 0.65 | 2:1 |
| Example 12 | 150 | 12 | 50 | 0.65 | 3:1 |
| Example 13 | 200 | 16 | 50 | 0.65 | 4:1 |
| Example 14 | 250 | 20 | 50 | 0.65 | 5:1 |
| Example 15 | 300 | 24 | 50 | 0.65 | 6:1 |
| Example 16 | 350 | 28 | 50 | 0.65 | 7:1 |
| Example 17 | 400 | 32 | 50 | 0.65 | 8:1 |
| Example 18 | 450 | 36 | 50 | 0.65 | 9:1 |
| Example 19 | 500 | 40 | 50 | 0.65 | 10:1 |

### Comparative Example 1: Aqueous solution of glycyrrhizic acid

10 mg of glycyrrhizic acid (93%, G810520-25g, Shanghai Macklin Biochemical Technology Co., Ltd.) was added to 16 mL of distilled water, evenly stirred, and subjected to ultrasonication for 10 minutes to obtain an aqueous solution of glycyrrhizic acid.

### Comparative Example 2: Aqueous bacterial cellulose

50 mg of the bacterial cellulose derived from *Acetobacterium Balch* (nanoscale lypophilized tablet of bacterial cellulose, Evophancie Biotech Ltd.) was added to 1.3 mL of distilled water, stirred thoroughly, and subjected to ultrasonication for 10 minutes to obtain an aqueous bacterial cellulose.

### Comparative Example 3: Baicalin bacterial cellulose

10 mg of baicalin (8802695-5g, Shanghai Macklin Biochemical Technology Co., Ltd.) was added to 0.8 mL of distilled water, stirred thoroughly, and subjected to ultrasonication for 10 minutes to obtain an aqueous solution of baicalin. Meanwhile, 100 mg of the bacterial cellulose derived from *Acetobacterium Balch* (nanoscale lypophilized tablet of bacterial cellulose, Evophancie Biotech Ltd.) was added to 1.3 mL of distilled water, stirred thoroughly, and subjected to ultrasonication for 10 minutes to obtain aqueous bacterial cellulose. Under ultrasonication, the aqueous solution of baicalin was added to the aqueous bacterial cellulose, stirred thoroughly, and subjected to ultrasonication for 15 minutes. The baicalin bacterial cellulose composite is obtained after rotary evaporation.

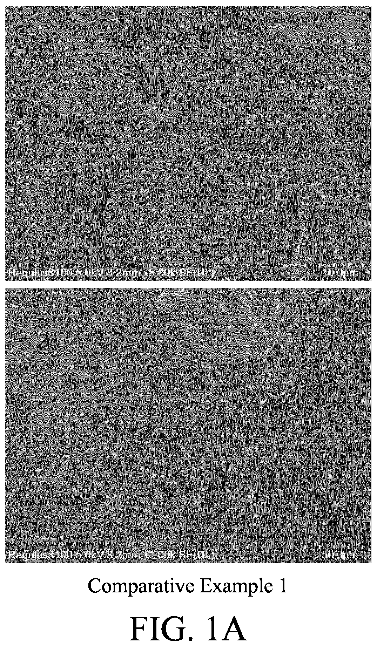

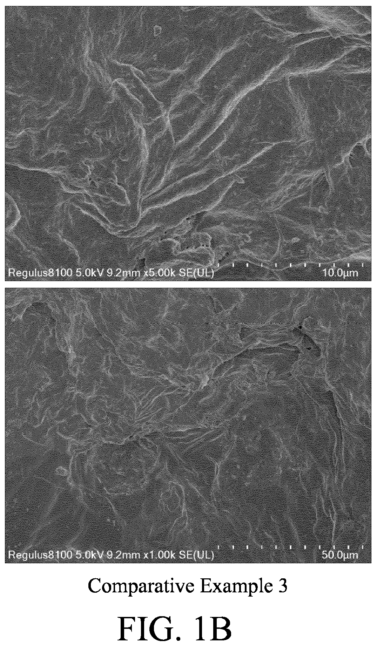

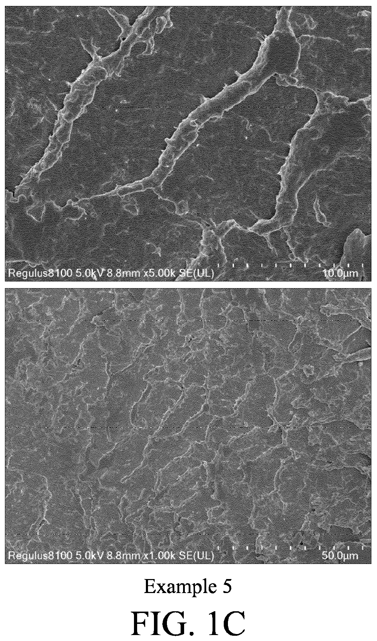

Please refer to FIGs. 1A-1C. Comparative Example 1, Comparative Example 3, and Example 5 were determined by a scanning electron microscope (FEI Quanta 400 FEI, America FEI scanning electron microscope), respectively, and the resulting SEM images were shown in FIGs. 1A-1C, respectively. As shown in FIGs. 1A-1C, only the glycyrrhizic acid bacterial cellulose composite prepared in Example 5 had a structure of glycyrrhizic acid packaged in the network of the bacterial cellulose.

In order to show the ability of glycyrrhizic acid in different samples (i.e., Comparative Examples 1-3 and Examples 1-19) on permeating skin, skin permeation experiments were performed with a Franz cell diffusion cell on back skin of SD rats. Firstly, prior to an experiment, the skin (3 cm x 3 cm) in the diffusion cell was cleaned with 50% ethanol, 200 mg of a sample was spread uniformly on the cleaned skin, and after the experiment, the skin was cleaned with 20% ethanol to remove the glycyrrhizic acid on surface of the skin. Absorbing solutions were collected after 2, 4 and 6 hrs, respectively, and 0.1 mL of the absorbing solution was mixed with 0.9 mL of methanol. Next, a high-performance liquid chromatography was performed on the mixture of the absorbing solution and methanol to determine the concentrations of glycyrrhizic acid in the skin over different time periods.

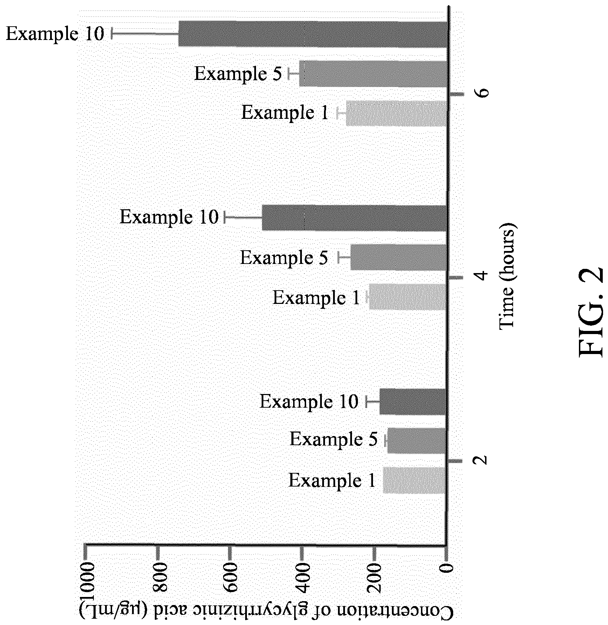

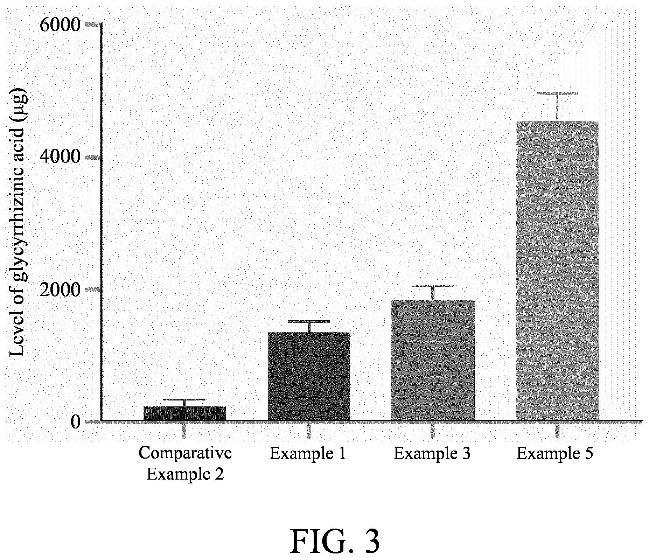

The skin permeation experiment and the high performance liquid chromatography aforementioned were performed by using Comparative Example 2, Example 1, Example 5 and Example 10 as the samples, respectively, each in triplicate and averaged, and the results were shown in FIG. 2. In addition, the skin permeation experiment and the high performance liquid chromatography aforementioned were performed by using Comparative Example 2, Example 1, Example 3, and Example 5 as the samples, respectively, to determine the levels of glycyrrhizic acid at the endpoint of skin permeation (i.e., 360 minutes after initiation of the experiment). As seen from the results shown in FIGs. 2 and 3, no presence of glycyrrhizic acid in the absorbing solution in Comparative Example 2 was detected; and as the content of bacterial cellulose in the glycyrrhizic acid bacterial cellulose composite increased, the additive permeating amount of glycyrrhizic acid increased, suggesting that glycyrrhizic acid in the glycyrrhizic acid bacterial cellulose composite containing a high proportion of bacterial cellulose had a greater ability of permeating skin.

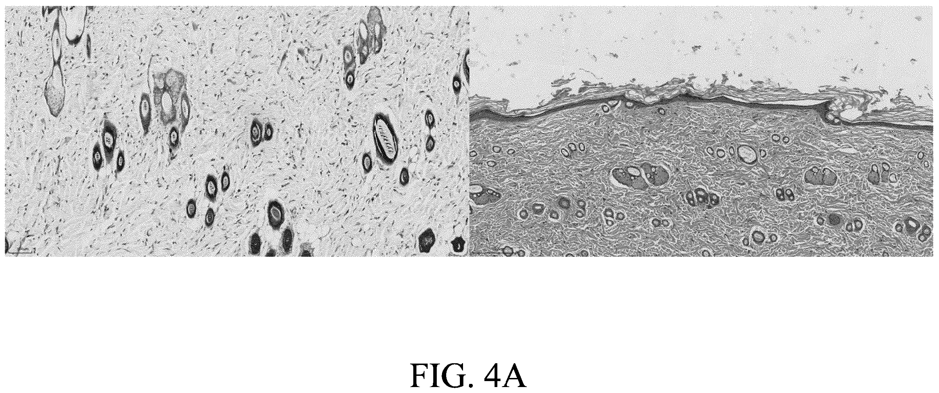

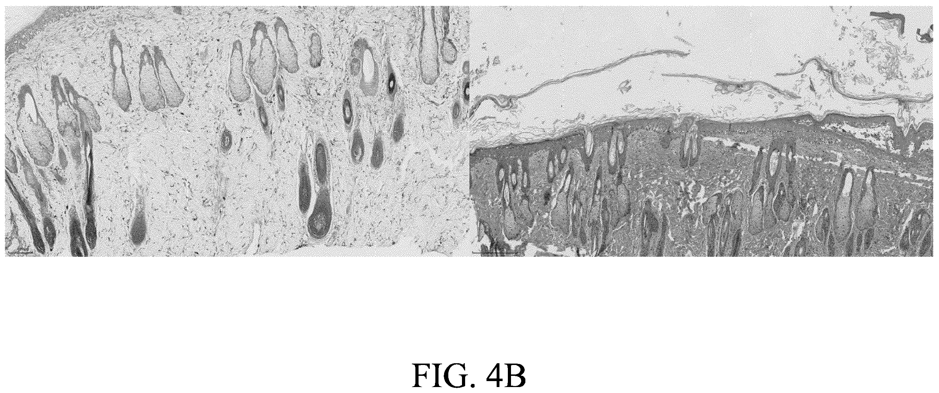

Please refer to FIGs. 4A and 4B. The skin was stained with toluidine blue (left panel) and HE staining (right) before performing the skin penetration experiment aforementioned and at the endpoint of skin penetration, respectively. The skin tissue untreated was compared to that treated with the glycyrrhizic acid bacterial cellulose composite of Example 10, and skins in equal area were observed for the number and morphological changes of hair follicles. As shown in FIGs. 4A and 4B, the glycyrrhizic acid bacterial cellulose composite provided in the present disclosure can promote the growth and development of hair follicles in skin.

In conclusion, the glycyrrhizic acid bacterial cellulose composite of the present disclosure has glycyrrhizic acid in a 3-dimensional network structure of bacterial cellulose, which can effectively facilitate the growth and development of hair follicles in skin. In addition, the glycyrrhizic acid bacterial cellulose composite provided in the present disclosure is easy for manufacturing, the ratio of glycyrrhizic acid to bacterial cellulose contained in the composite can be easily adjusted, and is in prospect of applying.

The examples described above are provided for the purpose of illustration only and not for limiting the present application. Modifications and alternations can be made to the examples described above by one skilled in the art without departing from the spirit and scope of the present application. Therefore, the claimed range of the present application is defined by the claims attached, and should be encompassed in the technical solutions of the present disclosure as long as it has no impact on the results and implementation of the present application.

## Claims

1. A glycyrrhizic acid bacterial cellulose composite for promoting growth and development of skin hair folliecles, comprising glycyrrhizic acid and bacterial cellulose.

2. The glycyrrhizic acid bacterial cellulose composite of claim 1, wherein the bacterial cellulose is derived from *Acetobacterium Balch* and has a molecular weight of 50,000 to 2,500,000.

3. The glycyrrhizic acid bacterial cellulose composite of claim 1, wherein the bacterial cellulose has 300-15,000 glucosyl groups.

4. The glycyrrhizic acid bacterial cellulose composite of claim 1, wherein a mass ratio of the glycyrrhizic acid to the bacterial cellulose is 1:0.1 to 10.

5. The glycyrrhizic acid bacterial cellulose composite of claim 1, wherein a mass ratio of the glycyrrhizic acid to the bacterial cellulose is 1:5.

6. A method for preparing the glycyrrhizic acid bacterial cellulose composite of claim 1, comprising steps of:
mixing water and a lyophilized bacterial cellulose to obtain an aqueous bacterial cellulose;
dissolving glycyrrhizic acid in water to obtain an aqueous solution of glycyrrhizic acid; and
adding the aqueous solution of glycyrrhizic acid dropwise to the aqueous bacterial cellulose to obtain the glycyrrhizic acid bacterial cellulose composite.

7. The method of claim 6, wherein the aqueous bacterial cellulose and the aqueous solution of glycyrrhizic acid are each stirred to form homogeneous solutions.

8. The method of claim 7, wherein the stirred aqueous bacterial cellulose and the stirred aqueous solution of glycyrrhizic acid are each further subjected to an ultrasonic treatment.

9. The method of claim 6, wherein the adding is performed under ultrasonication.

10. The method of claim 6, wherein the lyophilized bacterial cellulose and water are mixed in a weight ratio of 1: 10-15.

11. The method of claim 6, wherein the glycyrrhizic acid and water are mixed in a weight ratio of 1:0.5-1.5.
